# EUROPÄISCHE PATENTSCHRIFT

(11) **EP 0 808 821 B1**
(45) Veröffentlichungstag und Bekanntmachung des Hinweises auf die Patenterteilung: **23.08.2000**
(21) Anmeldenummer: 97107126.1
(22) Anmeldetag: 30.04.1997
(51) Int. Cl.: C07C 68/02, C07C 69/96

(54) **Verfahren zur kontinuierlichen Herstellung von Arylcarbonaten**
Process for the continuous preparation of aryl carbonates
Procédé de fabrication continue de carbonates d'aryle

(30) Priorität: 13.05.1996 DE 19619224
(43) Veröffentlichungstag der Anmeldung: 26.11.1997
(73) Patentinhaber: BAYER AG, 51368 Leverkusen (DE)
(72) Erfinder: Ooms, Pieter, Dr., 47800 Krefeld (DE); Bischof, Eric, Dr., 77520 Baytown Texas (US); Buysch, Hans-Josef, Dr., 47809 Krefeld (DE); Kühling, Steffen, Dr., 40670 Meerbusch (DE); Zaby, Gottfried, Dr., 51375 Leverkusen (DE); Jakob, Wolfgang, Dipl.Ing., 47447 Moers (DE); Dahmer, Jürgen, Dr., 47800 Krefeld (DE); Schön, Norbert, Dr., 97070 Würzburg (DE)

(56) Entgegenhaltungen:
- EP-A- 0 483 632
- EP-A- 0 635 476

## Beschreibung

Die vorliegende Erfindung betrifft ein Verfahren zur kontinuierlichen Herstellung von Carbonaten mit aromatischen Estergruppen durch Umsetzung von aromatischen Hydroxyverbindungen mit Phosgen in der Gasphase in Gegenwart von heterogenen Katalysatoren.

Es ist bekannt, daß Arylcarbonate durch Phasengrenzflächenphosgenierung (Schotten-Baumann-Reaktion) von aromatischen Hydroxyverbindungen gewonnen werden können. Dabei wirkt sich die Verwendung von Lösungsmitteln und Natronlauge nachteilig aus, da durch die wässrige Lauge eine teilweise Verseifung von Phosgen oder Chlorkohlensäureester stattfinden kann, große Mengen Kochsalz als Nebenprodukt anfallen und das Lösungsmittel zurückgewonnen werden muß.

Vorschläge für Verfahren ohne Lösungsmittel finden sich z.B. in US-A 2 837 555; 3 234 263; 2 362 865. Es werden jedoch lösliche Katalysatoren verwendet, deren Abtrennung von den Produkten aufwendig ist.

Somit erscheint es sinnvoll, heterogene, nicht lösliche Katalysatoren zu verwenden, die eine Aufarbeitung des Reaktionsgemisches wesentlich erleichtern. Auch dazu wurden Vorschläge gemacht. So wird in der EP-A-516 355 vor allem Aluminiumtrifluorid empfohlen, das gegebenenfalls auf Träger wie Alumosilikate aufgebracht wird. Die Synthese von Aluminiumfluorid ist jedoch durch die Handhabung von Fluor oder Flußsäure sehr aufwendig und teurer. In der WO 91/06526 werden Metallsalze auf porösen Trägern als Katalysatoren für eine vollkontinuierliche Phosgenierung von Phenol in der Gas- und Flüssigphase beschrieben. Die partielle Kondensation von Diphenylcarbonat bringt die Gefahr eines Auswaschens der aktiven Katalysatorbestandteile von den Trägern und somit einer Desaktivierung mit sich.

Nachteilig sind auch die unbefriedigende Umsätze selbst bei großem Phenol- oder Phosgenüberschuß, während im Falle eines Phosgenüberschusses erhebliche Anteile des unerwünschten Chlorameisensäurephenylesters erhalten werden, was zu einer aufwendigen Aufarbeitung führen würde.

Bisher gibt es also noch keinen Lösungsvorschlag für eine kontinuierliche technische Verfahrensweise zur Herstellung von Diarylcarbonaten durch Phosgenierung von aromatischen Hydroxyverbindungen in der Gasphase in Gegenwart von einheitlichen heterogenen Katalysatoren.

Ein solches Verfahren wurde nun gefunden. Es ist gekennzeichnet dadurch, daß man
1) ein auf Temperaturen von 180°C bis 500°C erhitztes, gasförmiges Gemisch aus aromatischer Hydroxyverbindung und gegebenenfalls deren Chlorameisensäureester, gegebenenfalls verdünnt mit einem Inertgas oder mit den Dämpfen eines inerten Lösungsmittels einerseits, und auf Temperaturen von 20°C bis 500°C erhitztes Phosgen andererseits in einen mit heterogenem Katalysator gefüllten, auf 180°C bis 500°C erhitzten, zylindrischen Reaktionsraum ohne sich bewegende Teile einleitet und darin reagieren läßt, wobei die Reaktionstemperatur um maximal 150°C über die Eintrittstemperatur des Reaktionsgemisches steigt,
2) das den Reaktor verlassende Gasgemisch partiell kondensiert, das Abgas zusammen mit einem gasförmigen oder schmelzflüssigen Strom der aromatischen Hydroxyverbindung, der gegebenenfalls etwas Chlorameisensäureester enthält, in einen mit heterogenem Katalysator gefüllten Abgasnachreaktor einleitet und darin so reagieren läßt, daß Phosgen und gegebenenfalls Chlorameisensäureester aus dem Abgasstrom entfernt werden,
3) das aus dem Reaktor austretende kondensierte und entgaste Reaktionsprodukt entweder direkt der Aufarbeitung zuführt oder aber in einen Nachreaktor einspeist, in dem restlicher Chlorameisensäureester über heterogenem Katalysator mit noch vorhandender oder zugespeister aromatischer Hydroxyverbindung weiter zu Diarylcarbonat umgesetzt wird,
4) das den Nachreaktor verlassende Produkt wiederum entgast und dieses Abgas dem unter 2) genannten Abgasnachreaktor mit schmelzflüssiger oder gasförmiger aromatischer Hydroxyverbindung zuführt,
5) das entgaste Produkt aus dem Nachreaktor in eine Destillationskolonne einspeist, aromatische Hydroxyverbindung und gegebenenfalls noch vorhandene Spuren Chlorameisensäureester über Kopf abdestilliert und in den Abgasnachreaktor oder in den ersten Reaktor wieder einleitet,
6) den Sumpf dieser ersten Kolonne einer zweiten Destillationskolonne zuleitet, in ihr über Kopf gegebenenfalls noch vorhandene Spuren Leichtsieder aus dem Diarylcarbonat entfernt, die in den oberen Teil der ersten Kolonne zurückgegeben werden,
7) aus dem Gasraum der zweiten Kolonne reines Diarylcarbonat, gegebenenfalls mit Spuren Hochsieder, ausspeist,
8) dieses Produkt einer dritten Kolonne zuführt und durch Abtrennung von Restmengen Hochsieder als Sumpf reines Diarylcarbonat als Kopfprodukt erhält,
9) die vereinigten Sümpfe der zweiten und dritten Kolonne einer vierten Destillationseinheit zuführt, über Kopf Diarylcarbonat abdestilliert, es in die zweite Kolonne zurückleitet und aus dem Sumpf der vierten Destillationseinheit die Hochsieder entnimmt.

Aromatische Hydroxyverbindungen für das erfindungsgemäße Verfahren sind solche der Formel

ArOH,

worin
- Ar: Phenyl, Naphthyl, Anthryl, Phenanthryl, Indanyl, Tetrahydronaphthyl oder den Rest eines 5- oder 6-gliedrigen aromatischen Heterocyclus mit 1 oder 2 Heteroatomen aus der Gruppe von N, O und S bedeutet, wobei diese isocyclischen und heterocyclischen Reste durch einen oder mehrere Substituenten wie geradkettige oder verzweigte C₁-C₄-Alkyl-, C₁-C₄-Alkenyl-, C₁-C₄-Alkoxygruppen, Phenylreste oder Nitril- und Halogenfunktionen substituiert sein können, und wobei weiterhin die heterocyclischen Reste mit einem ankondensierten Benzolkern verbunden sein können.

Beispiele für erfindungsgemäße aromatische Hydroxyverbindungen sind: Phenol, o-, m- und p-Kresol, o-, m- und p-Isopropylphenol, die entsprechenden Halogen- bzw. Alkoxyphenole, wie p-Chlorphenol bzw. p-Methoxyphenol, ferner Monohydroxyverbindungen des Naphthalins, Anthracens und Phenanthrens, weiterhin 4-Hydroxypyridin und Hydroxychinoline. Vorzugsweise werden substituierte Phenole, ganz besonders bevorzugt Phenol selbst eingesetzt.

Die aromatischen Hydroxyverbindungen und deren Chlorameisensäureester werden vor der Durchführung des erfindungsgemäßen Verfahrens verdampft und kontinuierlich auf eine Temperatur innerhalb des Temperaturbereichs von 180 bis 500°C, vorzugsweise 200 bis 400°C, besonders bevorzugt 220 bis 350°C erhitzt. Die zuvor erhitzten aromatische Hydroxyverbindungen können beim erfindungsgemäßen Verfahren als solche oder in mit Inertgas oder mit Dämpfen eines inerten Lösungsmittels verdünnter Form zum Einsatz gelangen. Die Durchmischung der Dämpfe der aromatischen Hydroxyverbindung mit dem Inertgas kann beispielsweise durch Verdampfen der aromatischen Hydroxyverbindung im Strom eines inerten Gases oder der Dämpfe eines inerten Lösungsmittels erfolgen. Bevorzugtes Inertgas ist Stickstoff. Geeignete inerte Lösungsmittel, deren Dämpfe ebenfalls zur Verdünnung der aromatischen Hydroxyverbindungen verwendet werden können, sind beispielsweise Chlorbenzol, o-Dichlorbenzol, Xylol, Chlornaphthalin, Decahydronaphthalin oder deren Gemische.

Die Menge des gegebenenfalls als Verdünnungsmittel mitverwendeten Inertgases oder Lösungsmitteldampfes ist nicht kritisch.

Geeignete Katalysatoren für das erfindungsgemäße Verfahren sind bekannt, beispielsweise aus EP-A 483 632, EP-A 635 476, US-A 5 478 961, EP-A 635 477, US-A 5 473 094, EP-A 645 364, US-A 5 524 942, EP-A 691 326, EP-A 722 930, EP-A 516 355, US-A 5 239 105 und US-A 5 136 077.

Die Edukte Phosgen und Hydroxyverbindung setzt man in Molverhältnissen von 1:0,5 bis 1:8, bevorzugt 1:1,5 bis 1:5, besonders bevorzugt 1:2 bis 1:4 ein. Das stöchiometrische Verhältnis ist in diesem Fall 1:2.

Die Katalysatoren werden in der Regel als körniges Material, Granulate, Extrudate, Stäbchen, Kugeln, oberflächenreiche Formkörper wie Hohlextrudate in Form von Raschigringen, Hohlzylindern, Sternen, Wagenrädern oder als Bruchstücke eingesetzt. Durchmesser und Länge dieser Teilchen betragen 0,5 bis 10 mm. Sie werden im Reaktor in Form einfacher Schüttungen angeordnet.

Vor der Durchführung des erfindungsgemäßen Verfahrens wird der Phosgenstrom auf eine Temperatur innerhalb des Bereiches von 20 bis 500°C, vorzugsweise 100 bis 400°C, besonders bevorzugt von 220 bis 350°C erhitzt.

Zur Durchführung der erfindungsgemäßen Umsetzung werden die vorerhitzten Ströme der aromatischen Hydroxyverbindung bzw. des Gemisches aus aromatischer Hydroxyverbindung und Inertgas oder Dämpfen eines inerten Lösungsmittels einerseits und des Phosgens andererseits kontinuierlich in einen zylindrischen Reaktionsraum geleitet und dort miteinander vermischt.

Geeignete Reaktoren für das erfindungsgemäße Verfahren sind dem Fachmann bekannt. Beispiele sind Rohrreaktoren ohne sich bewegende Teile im Innern des Reaktors, gegebenenfalls mit Kühl- bzw. Heizmantel, die den Katalysator als Schüttung enthalten, oder Hordenreaktoren, in denen der Katalysator als gleichmäßige Schicht auf mehrere übereinanderliegende Böden verteilt ist.

Die Rohrreaktoren bestehen im allgemeinen aus Stahl, Glas, legiertem oder emailliertem Stahl und weisen eine Länge auf, die ausreichend ist, um unter den Verfahrensbedingungen eine fast vollständige Umsetzung der aromatischen Hydroxyverbindung mit dem Phosgen zu ermöglichen. Die Gasströme werden im allgemeinen an einem Ende des Rohrreaktors eingeleitet, wobei diese Einleitung beispielsweise durch an einem Ende des Rohrreaktors angebrachte Düsen oder durch eine Kombination aus Düse und einem Ringspalt zwischen Düse und Mischrohr oder durch einen Verteilerboden erfolgen kann.

Die Umsetzung von Phosgen und aromatischer Hydroxyverbindung wird durchgeführt bei Temperaturen von etwa 180 bis 500°C, vorzugsweise 200 bis 400°C, besonders bevorzugt 220 bis 350°C.

Der Druck bewegt sich im Bereich von 0,1 bis 10 bar, bevorzugt 0,2 bis 7 bar, besonders bevorzugt von 0,3 bis 6 bar.

Die Strömung durch den Reaktor kann durch Einstelllung eines entsprechenden Differenzdrucks zwischen den Produktzuleitungen zum Reaktionsraum einerseits und dem Ausgang aus dem Reaktionsraum andererseits sichergestellt werden. Im allgemeinen liegt der Druck in den Zuleitungen zum Reaktionsraum bei 200 bis 3000 mbar und am Ausgang aus dem Reaktionsraum bei 150 bis 2000 mbar. Wesentlich ist hierbei jedoch lediglich die Aufrechterhaltung eines Differenzdrucks zwecks Gewährleistung der Durchströmung des Reaktorraumes. Das den Reaktionsraum verlassende gasförmige Gemisch wird in der Regel abgekühlt und der Aufarbeitung zugeführt oder teilkondensiert und einem Nachreaktor zugeleitet.

Die Teilkondensation kann gegebenenfalls mit Hilfe eines inerten Lösungsmittels erfolgen, dessen Temperatur so gewählt wird, daß das Diarylcarbonat und ein gegebenenfalls als Verdünnungsmittel mitverwendetes Lösungsmittel kondensieren bzw. sich in dem inerten Lösungsmittel lösen, während überschüssiges Phosgen, Chlorwasserstoff und gegebenenfalls als Verdünnungsmittel mitverwendetes Inertgas die Kondensationsstufe bzw. das inerte Lösungsmittel gasförmig durchlaufen. Beispiele für die selektive Kondensation sind das Durchleiten des Gasgemisches durch das inerte Lösungsmittel oder das Eindüsen des Lösungsmittels (Lösungsmittelnebel) in den Gasstrom.

Geeignete Lösungsmittel, die zur selektiven Kondensation verwendet werden können, sind beispielsweise Chlorbenzol, o-Dichlorbenzol, Xylol, Chlornaphthalin, Decahydronaphthalin oder deren Gemische, bevorzugt Dichlorbenzol sowie besonders bevorzugt die aromatische Hydroxyverbindung selbst.

Grundsätzlich kann die Abkühlung bzw. Teilkondensation jedoch auch mit einem Wärmetauscher durchgeführt werden.

Das nach der Teilkondensation verbleibende Abgas wird durch einen mit heterogenem Katalysator gefüllten Reaktor zusammen mit einem schmelzflüssigem oder gasförmigen Strom der Hydroxyverbindung, der auch Chlorameisensäureester in Mengen von <50 Gew.-%, vorzugsweise <30 Gew.- %, besonders bevorzugt <10 Gew.-% enthalten kann, in Gleich- oder Gegenstrom geleitet, wobei dem Abgasstrom restliches Phosgen und gegebenenfalls noch mitgeführte kleinere Mengen an Chlorameisensäureester entzogen werden. Das den Abgasnachreaktor verlassende schmelzflüssige Gemisch wird durch Zugabe von gegebenenfalls weiterer Hydroxyverbindung auf das gewünschte Molverhältnis eingestellt, verdampft, auf die gewünschte Temperatur erhitzt und dem Reaktor zugeführt.

Der nach Entgasung des Reaktionsgemisches aus dem Abgasnachreaktor am Kopf austretende Gasstrom besteht im wesentlichen aus Chlorwasserstoff. Noch eventuell vorhandenes Phosgen kann nach bekannten Methoden in einem Aktivkohleturm mit wenig Wasser hydrolysiert werden. Die entsprechend ihrem Dampfdruck bei der in der Entgasungsapparatur herrschenden Temperatur noch im Chlorwasserstoffstrom befindliche Menge der Hydroxyverbindung wird durch Ausfrieren in einer Kühlfalle entfernt und kann in den Reaktor zurückgeführt werden. Eine eventuelle Restmenge wird in der nachfolgenden adiabatischen Absorption des Chlorwasserstoffes in Wasser durch azeotrope Destillation als wäßrige Mischung ausgetrieben und kann nach Wiedergewinnung dem Reaktor zugeführt oder aber für andere Zwecke, wie der Herstellung von Phenolharzen, verwendet werden.

Die restlichen Phosgenmengen, die sich noch im Chlorwasserstoff befinden, können auch vorteilhaft nach der adiabatischen Absorption mit Wasser, wo sie mit dem Azeotrop aus Hydroxyverbindung und Wasser ausgetrieben werden, mit den restlichen aus den Eduktströmen stammenden Inertgasspuren dem Aktivkohleturm zugeführt und darin hydrolysiert werden.

Nach der partiellen Kondensation des Reaktionsgemisches erhält man ein erstes Rohprodukt, das in der Regel überwiegend aus Diarylcarbonat und aromatischer Hydroxyverbindung besteht, und das noch gewisse Mengen an Chlorameisensäureester enthält, die in der Regel <50 Gew.-%, bevorzugt <30 Gew.-%, ganz besonders bevorzugt <15 Gew.-% betragen.

Dieses Gemisch kann man direkt der destillativen Aufarbeitung zuführen und in Ströme aus Chlorameisensäureester und Hydroxyverbindung, einem Diarylcarbonat und kleinen Mengen Hochsieder aufteilen. Zur Vereinfachung und ökonomischeren Durchführung der Destillation ist jedoch ein Reaktionsgemisch besser geeignet, das keinen oder nur kleine Mengen an Chlorameisensäureester enthält.

Daher leitet man vorteilhafterweise das nach der partiellen Kondensation erhaltene erste schmelzflüssige Rohprodukt in einen zweiten, heterogenen Katalysator enthaltenden Nachreaktor und setzt darin den noch vorhandenen Chlorameisensäureester mit im Gemisch noch befindlicher oder zugesetzter Hydroxyverbindung in flüssiger Phase um. Dabei kann der Druck in engeren Grenzen von 0,1 bis 6, vorzugsweise 0,2 bis 4 bar gehalten werden. Die Temperatur beträgt 120 bis 250°C, vorzugsweise 140 bis 250°C, besonders bevorzugt 160 bis 230°C.

Die Belastung der Reaktoren, gemessen in Kilogramm Eduktgemisch pro Liter Katalysatorvolumen und Stunde hängt von der Reaktionstemperatur, der Aktivität der Katalysatoren und dem gewünschten Umsatz ab. Sie beträgt 0,01 bis 20, vorzugsweise 0,02 bis 10, besonders bevorzugt 0,03 bis 4, ganz besonders bevorzugt 0,04 bis 3 kg/l·h.

Das den Nachreaktor verlassende Gemisch wird entgast und der Abgasstrom ebenfalls in den Abgasnachreaktor geleitet. Das entgaste Gemisch, das nur noch geringe Mengen (<3, bevorzugt <2, besonders bevorzugt <1 Gew.-%) Chlorameisensäureester enthält, wird in einer ersten Destillationskolonne von überschüssiger Hydroxyverbindung und Chlorameisensäurester oder gegebenenfalls mitverwendeten Lösungsmittel befreit, die als Kopfprodukt abgenommen und je nach Bedarf in den Reaktor, Nachreaktor oder in den Abgasnachreaktor geleitet und weiter umgesetzt werden.

Das am Boden dieser Kolonne ablaufende Gemisch wird in einer zweiten Kolonne in restliche Leichtsieder, die in den oberen Teil der ersten Kolonne zurückgeführt werden, reines Diarylcarbonat, das seitlich aus dem Dampfstrom dieser zweiten Kolonne entnommen wird, und in ein Gemisch aus Diarylcarbonat und Hochsieder, das die Kolonne als Sumpf verläßt, aufgetrennt.

Durch Einspeisung in eine dritte Kolonne wird das Diarylcarbonat, das als Kopfprodukt dieser dritten Kolonne entnommen wird, von Spuren Hochsieder (Sumpf) getrennt.

Die vereinigten Sümpfe aus der zweiten und dritten Kolonne trennt man in einer vierten Destillationsvorrichtung, die kontinuierlich oder diskontinuierlich betrieben wird, in einen die Hochsieder enthaltenden Sumpf und Diarylcarbonat auf, das in den unteren Teil der zweiten Kolonne geleitet und dort weiter gereinigt wird.

Die vergleichsweise geringen Mengen Sumpf von <3, vorzugsweise <2, besonders bevorzugt <1 % des Reaktionsproduktes werden zweckmäßig verbrannt oder zur Phenolharzherstellung eingesetzt.

### Beispiel

### Kontinuierliche Herstellung von Diphenylcarbonat durch Gasphasen-Phosgenierung von Phenol in Gegenwart von γ-Aluminiumoxid

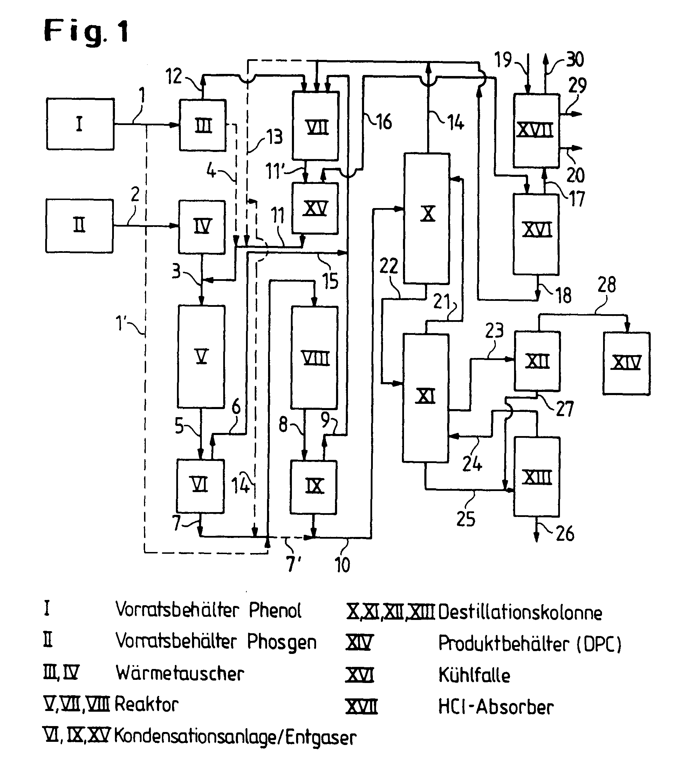

Die zur Durchführung des erfindungsgemäßen Verfahrens eingesetzte Apparatur und die auftretenden Stoffströme sind schematisch in Figur 1 wiedergegeben.

Zusammen mit aus der Destillationskolonne X am Kopf entnommenem Phenol 14 und aus der Kühlfalle XVI zurückgeführtem Phenol 18 dosiert man aus einem beheizten Behälter I über Wärmetauscher III (170°C) unter Normaldruck 54,0 Gew.-Teile/h Phenol 12 von oben in einen auf 170°C beheizten, mit 45 Vol.-Teilen γ-Aluminiumoxid gefüllten Abgasnachreaktor VII. In Gleichstrom damit wird der aus der Kondensationsapparatur VI und der Entgasungsapparatur IX stammende Abgasstrom 15 (Gewichtsverhältnis Phenol/Phosgen/Chlorwasserstoff/ Kohlendioxid 1,7/52,9/44,8/0,6) in Reaktor VII eingespeist.

Das am Fuß des Reaktors austretende Produkt 11', das Phenol, Chlorameisensäurephenylester, Diphenylcarbonat und Nebenprodukte im Verhältnis 82,0/1,35/ 16,6/0,05 enthält, wird über Entgaser XV in Abgas 16 (Gewichtsverhältnis Phenol, Phosgen, Chlorwasserstoff und Kohlendioxid 4,0/66,3/29,5/0,2) und Sumpf 11 (Gewichtsverhältnis Phenol, Chlorameisensäurephenylester, Diphenylcarbonat und Nebenprodukte 81,9/1,4/16,7/0,05) aufgetrennt.

40,6 Gew.-Teile/h über Wärmetauscher IV (280°C) vorgeheiztes Phosgen 3 wird zusammen mit auf 280°C vorgeheiztem 11 in einem auf 280°C beheizten, mit 45 Vol.-Teilen γ-Aluminiumoxid gefüllten Reaktor V in Gleichstrom eingeleitet.

Das am Fuß des Reaktors austretende Produkt 5, das Phenol, Chlorameisensäurephenylester, Diphenylcarbonat und Nebenprodukte im Verhältnis 34,1/4,3/61,3/0,3 enthält, wird über Kondensationsapparatur VI in Abgas 6 (Gewichtsverhältnis Phenol, Phosgen, Chlorwasserstoff und Kohlendioxid 0,9/54,8/43,7/0,6) und Sumpf 7 (Gewichtsverhältnis Phenol, Chlorameisensäurephenylester, Diphenylcarbonat und Nebenprodukte 33,9/4,3/61,5/0,3) aufgetrennt.

Der im Sumpf 7 vorhandene Chlorameisensäurephenylester wird durch Nachreaktion mit vorhandenem Phenol (eventuell nach Zugabe von zusätzlichem Phenol (1' oder 14') in einem Nachreaktor VIII, ebenfalls befüllt mit γ-Aluminiumoxid (45 Vol.-Teile) bei 180°C zu Diphenylcarbonat umgesetzt.

Das am Reaktorfuß entnommene Produkt 8 (Gewichtsverhältnis Phenol, Diphenylcarbonat und Nebenprodukte 31,7/68,0/0,3) wird über Entgaser IX in Abgas 9 (Phenol/Chlorwasserstoff) und Sumpf 10 (Gewichtsverhältnis Phenol, Diphenylcarbonat und Nebenprodukte 31,5/68,2/0,3) aufgetrennt.

Abgasströme 6 und 9 werden vereinigt zu 15 und in Abgasnachreaktor VII in Gleichstrom mit Phenol (12, 14 und 18) geleitet, wie oben bereits erwähnt.

Das am Kopf des Entgasers XV austretende Abgas 16 wird einer Kühlfalle XVI zugeleitet, in der vorhandenes Phenol 18 (0,70 Gew.-Teile/h) entfernt und in Abgasnachreaktor VII zurückgegeben wird.

Das aus der Kühlfalle XVI austretende Abgas 17 wird einer Chlorwasserstoffabsorptionsanlage XVII zugeführt.

Durch Einspeisung von 124,4 Gew.-Teilen/h einer 18 %igen Salzsäure 19 erhält man 145,7 Gew.-Teile/h einer 30 %igen Salzsäure 20, die der Elektrolyse zugeführt werden kann. Das aus der Elektrolyse gewonnene Chlor kann wieder für die Herstellung von Phosgen verwendet werden.

Spuren mitgeführtes Phenol können als Azeotrop mit Wasser 29 entfernt werden.

Zur Zersetzung noch vorhandenen Phosgens im Abgas 30 wird eine Vernichtungsanlage (Aktivkohletürme mit Wasser) angeschlossen.

Sumpf 10 wird in eine erste Destillationskolonne X eingespeist und bei ca. 80°C/16 hPa (12 mm) aufgetrennt in 28,1 Gew.-Teile/h Phenol und Sumpf 22 (Gewichtsverhältnis Phenol, Diphenylcarbonat und Nebenprodukte 0,3/99,3/0,4).

Sumpf 22 wird einer zweiten Destillationskolonne XI zugeleitet, in der über Kopf noch vorhandenes Phenol 21 (0,2 Gew.-Teile/h) entfernt und in den oberen Teil der ersten Kolonne X zurückgegeben wird.

Durch seitliche Ausspeisung aus dem Gasraum der zweiten Kolonne XI erhält man 61,3 Gew.-Teile/h Produkt 23 (Gewichtsverhältnis Diphenylcarbonat, Nebenprodukte 99,9/0,1), das in einer dritten Destillationskolonne XII in Kopfprodukt 28 (61,3 Gew.-Teile/h Diphenylcarbonat) und Sumpf 27, der in Kolonne XIII fließt, aufgetrennt wird.

Das am Fuß der Destillationskolonne XI entnommene Produkt 25 (Gewichtsverhältnis Diphenylcarbonat/Nebenprodukte 90,6/9,4) wird zusammen mit 27 in einer vierten Destillationskolonne XIII bei 170°C/16 hPa (12 mm) in Kopfprodukt 24 (1,8 Gew.-Teile/h Diphenylcarbonat), das in den unteren Teil der zweiten Kolonne XI zurückgeführt wird, und Sumpf 26 (hochsiedende Nebenprodukte) getrennt.

### Beispiel 2

Durchführung des erfindungsgemäßen Verfahrens wie in Beispiel 1 beschrieben aber ohne Abgasnachreaktor und bei 330°C unter Einspeisung von 71,9 Gew.-Teilen/h Phenol 4 über Wärmetauscher III und Einleitung von 37,1 Gew.-Teilen/h über Wärmetauscher IV vorgeheiztem Phosgen 3 in Reaktor V, führt mit gleichbleibender Selektivität zu 26,1 Gew.-Teilen/h Diphenylcarbonat.

Als weitere Variationen zu der beschriebenen Verfahrensweise sind in Abhängigkeit von den Edukt- und Produktzusammensetzungen, Katalysatorbelastungen und Temperatur zu nennen:
a) Dosierung von Phenol 4 direkt in Reaktor V statt über Abgasnachreaktor VII beim Anfahren.
b) Zugabe von Phenol (1' oder 14') als zusätzlicher Reaktionspartner für die Nachreaktion bei Vorliegen größerer Konzentrationen an Chlorameisensäurephenylester.
c) Fahrweise ohne Nachreaktion im Reaktor VIII, wobei dann noch vorhandener Chlorameisensäurephenylester in der ersten Destillationskolonne X als Leichtsieder 14 mit Phenol abdestilliert und in Abgasnachreaktor VII eingespeist wird, oder bei einer Fahrweise ohne Abgasnachreaktor in den ersten Reaktor V zurückgeführt wird (13).
d) Fahrweise mit Phosgenwäsche statt Nachreaktor VII und Entgaser XV, wobei Abgasstrom 15 von unten in einer Gegenstromapparatur schmelzflüssigem Phenol (1, 14 oder 18) entgegengeschickt wird.

## Patentansprüche

1. Verfahren zur kontinuierlichen Herstellung von Diarylcarbonaten durch Umsetzung von Phosgen mit aromatischen Hydroxyverbindungen in Gegenwart von heterogenen Katalysatoren, dadurch gekennzeichnet, daß man
1) ein auf Temperaturen von 180°C bis 500°C erhitztes gasförmiges Gemisch aus aromatischer Hydroxyverbindung und gegebenenfalls deren Chlorameisensäureester, gegebenenfalls verdünnt mit einem Inertgas oder mit den Dämpfen eines inerten Lösungsmittels einerseits, und auf Temperaturen von 20°C bis 500°C erhitztes Phosgens andererseits in einen mit heterogenem Katalysator gefüllten, auf 180°C bis 500°C erhitzten, zylindrischen Reaktionsraum ohne sich bewegende Teile einleitet und darin reagieren läßt, wobei die Reaktionstemperatur um maximal 150°C über die Eintrittstemperatur des Reaktionsgemisches steigt,
2) das den Reaktor verlassende Gasgemisch partiell kondensiert, das Abgas zusammen mit einem gasförmigen oder schmelzflüssigen Strom der aromatischen Hydroxyverbindung, der gegebenenfalls etwas Chlorameisensäureester enthält, in einen mit heterogenem Katalysator gefüllten Abgasnachreaktor einleitet und darin so reagieren läßt, daß Phosgen und gegebenenfalls Chlorameisensäureester aus dem Abgasstrom entfernt werden,
3) das aus dem Reaktor austretende und entgaste Reaktionsprodukt entweder direkt der Aufarbeitung zuführt oder aber in einen Nachreaktor einspeist, in dem restlicher Chlorameisensäureester über heterogenen Katalysator mit noch vorhandener oder zugespeister aromatischer Hydroxyverbindung weiter zu Diarylcarbonat umgesetzt wird,
4) das den Nachreaktor verlassende Produkt wiederum entgast, und dieses Abgas dem unter 2) genannten Abgasnachreaktor mit schmelzflüssiger oder gasförmiger aromatischer Hydroxyverbindung zuführt,
5) das entgaste Produkt aus dem Nachreaktor in eine Destillationskolonne einspeist, aromatische Hydroxyverbindung und gegebenenfalls noch vorhandene Chlorameisensäureester über Kopf abdestilliert und in den Abgasnachreaktor oder in den ersten Reaktor wieder einleitet,
6) den Sumpf dieser ersten Kolonne einer zweiten Destillationskolonne zuleitet, in ihr über Kopf gegebenenfalls noch vorhandene Spuren Leichtsieder aus dem Diarylcarbonat entfernt, die in den oberen Teil der ersten Kolonne zurückgegeben werden,
7) aus dem Gasraum der zweiten Kolonne Diarylcarbonat, gegebenenfalls mit Spuren Hochsieder, ausspeist,
8) dieses Produkt einer dritten Kolonne zuführt und durch Abtrennung von Restmengen Hochsieder als Sumpf reines Diarylcarbonat als Kopfprodukt erhält,
9) die vereinigten Sümpfe der zweiten und dritten Kolonne einer vierten Destillationseinheit zuführt, über Kopf Diarylcarbonat abdestilliert, es in die zweite Kolonne zurückleitet und aus dem Sumpf der vierten Destillationseinheit die Hochsieder entnimmt.

## Claims

1. Process for the continuous production of diaryl carbonates by reacting phosgene with aromatic hydroxy compounds in the presence of heterogeneous catalysts, characterised in that
1) a gaseous mixture heated to 180°C to 500°C of an aromatic hydroxy compound and optionally the chloroformic acid ester thereof, optionally diluted with an inert gas or with the vapour of an inert solvent, on the one hand, and phosgene heated to 20°C to 500°C, on the other, are introduced into a cylindrical reaction chamber without moving parts, filled with heterogeneous catalyst and heated to 180°C to 500°C and allowed to react therein, wherein the reaction temperature rises by at most 150°C above the inlet temperature of the reaction mixture,
2) the gas mixture leaving the reactor is partially condensed, the exit gas is introduced together with a gaseous or molten stream of the aromatic hydroxy compound, which optionally contains some chloroformic acid ester, into an exit gas post-reactor filled with heterogeneous catalyst and is allowed to react therein in such a manner that phosgene and optionally chloroformic acid ester are removed from the exit gas stream,
3) the degassed reaction product leaving the reactor is either passed directly for working up or introduced into a post-reactor, in which any remaining chloroformic acid ester is further reacted by means of a heterogeneous catalyst with any aromatic hydroxy compound still present or introduced to yield diaryl carbonate,
4) the product leaving the post-reactor is again degassed and this exit gas is introduced into the exit gas post-reactor stated in 2) together with the molten or gaseous aromatic hydroxy compound,
5) the degassed product from the post-reactor is introduced into a distillation column, the aromatic hydroxy compound and any traces of chloroformic acid ester optionally still present are distilled off as a top product and reintroduced into the exit gas post-reactor or into the first reactor,
6) the bottom product of this first column is introduced into a second distillation column, in which any traces of low-boiling components optionally still present are removed from the diaryl carbonate as a top product and returned to the upper part of the first column,
7) diaryl carbonate, optionally with traces of high-boiling components, is discharged from the gas space of the second column,
8) this product is introduced into a third column and pure diaryl carbonate is obtained as a top product by separation from the residual amounts of high-boiling components as the bottom product,
9) the combined bottom products from the second and third columns are passed into a fourth distillation unit, diaryl carbonate is distilled off as the top product, returned to the second column and the high-boiling components are removed from the bottom of the fourth distillation unit.

## Revendications

1. Procédé pour la préparation en continu de diarylcarbonates par mise en réaction de phosgène avec des composés hydroxylés aromatiques en présence de catalyseurs hétérogènes, caractérisé en ce que
1) on introduit, dans un espace réactionnel cylindrique exempt d'éléments mobiles, chauffé à une température de 180°C à 500°C et garni avec un catalyseur hétérogène, un mélange gazeux chauffé à des températures de 180°C à 500°C, d'un composé hydroxylé aromatique et le cas échéant de son ester chloroformique, le cas échéant dilué avec un gaz inerte ou avec les vapeurs d'un solvant inerte d'une part et de phosgène chauffé à des températures de 20°C à 500°C d'autre part, et on y laisse réagir le mélange, la température réactionnelle dépassant au maximum de 150°C la température d'entrée du mélange réactionnel,
2) on soumet à une condensation partielle le mélange gazeux quittant le réacteur; on introduit les gaz d'échappement de manière conjointe avec un courant gazeux ou en fusion du composé hydroxylé aromatique qui contient le cas échéant une petite quantité d'ester chloroformique, dans un réacteur monté à la suite destiné aux gaz d'échappement et garni avec un catalyseur hétérogène, et on les y laisse réagir de telle sorte que l'on élimine du courant de gaz d'échappement le phosgène et le cas échéant l'ester chloroformique,
3) soit on achemine directement au traitement le produit réactionnel quittant le réacteur et dont on a éliminé les produits volatils, soit on l'introduit dans un réacteur monté à la suite dans lequel on soumet à une réaction ultérieure, l'ester chloroformique résiduel par-dessus un catalyseur hétérogène avec le composé hydroxylé aromatique encore présent ou ajouté, pour obtenir un diarylcarbonate,
4) on élimine une nouvelle fois les produits volatils du produit quittant le réacteur monté à la suite et on achemine ces gaz d'échappement au réacteur monté à la suite mentionné sous 2) destiné aux gaz d'échappement avec le composé hydroxylé aromatique en fusion ou gazeux,
5) on introduit dans une colonne de distillation le produit dont on a éliminé les produits volatils et qui provient du réacteur monté à la suite, on sépare par distillation via la tête de la colonne le composé hydroxylé aromatique et l'ester chloroformique le cas échéant encore présent et on les réintroduit dans le réacteur monté à la suite destiné aux gaz d'échappement ou dans le premier réacteur,
6) on achemine le produit du bas de cette première colonne à une deuxième colonne de distillation; par la tête de cette dernière, on élimine du diarylcarbonate des traces le cas échéant encore présentes de la fraction légère que l'on renvoie dans la partie supérieure de la première colonne,
7) on évacue de l'espace réservé aux gaz de la deuxième colonne du diarylcarbonate le cas échéant avec des traces de la fraction lourde,
8) on achemine ce produit à une troisième colonne et, par séparation des quantités résiduelles de la fraction lourde à titre de produit de bas de colonne, on obtient du diarylcarbonate pur à titre de produit de tête,
9) on achemine à une quatrième unité de distillation les produits de bas de colonne combinés provenant de la deuxième et de la troisième colonne; via la tête, on sépare le diarylcarbonate par distillation; on le renvoie dans la deuxième colonne et on extrait la fraction lourde du produit de bas de colonne de la quatrième unité de distillation.
